# EUROPEAN PATENT APPLICATION

(11) **EP 3 543 353 A1**
(43) Date of publication of application: **25.09.2019**
(21) Application number: 19172280.0
(22) Date of filing: 23.09.2014
(51) Int. Cl.: C12Q 1/68

(54) **METHODS AND COMPOSITIONS RELATING TO CANCER THERAPY WITH DNA DAMAGING AGENTS**

(30) Priority: 23.09.2013 US 201361881331 P
(62) Divisional of application: 14845394.7
(71) Applicant: The University of Chicago, Chicago, IL 60637 (US)
(72) Inventor: PITRODA, Sean, P., Chicago, IL 60637 (US); WEICHSELBAUM, Ralph, R., Chicago, IL 60637 (US); CONNELL, Philip, P., Chicago, IL 60637 (US)
(74) Representative: Hiebl, Inge Elisabeth

(57) **Abstract**

Methods and compositions are provided for predicting efficacy of a DNA damaging agent in a cancer patient or treating a cancer patient with a DNA damaging agent after evaluating efficacy. In some embodiments, an algorithm is used to evaluate efficacy and treatment.

## Description

### CROSS-REFERENCE TO RELATED APPLICATIONS

This application claims the benefit of priority of U.S. Provisional Patent Application No. 61/881,331, filed September 23, 2013, which is hereby incorporated by reference in its entirety.

### BACKGROUND OF THE INVENTION

### I. Field of the Invention

The present invention relates generally to the fields of biology, chemistry and medicine. More particularly, it concerns methods and compositions relating to oncology and cancer treatment.

### II. Description of Related Art

Homologous recombination (HR) and non-homologous end-joining (NHEJ) are competing pathways that repair double-stranded DNA breaks (DSBs) generated by radiation and some chemotherapeutic drugs. HR also serves additional functions such as promoting cellular tolerance to DNA-damaging drugs that disrupt replication forks (Thompson, et al., 2001). Both HR and NHEJ facilitate DNA repair following the recruitment of upstream sensor/effector proteins. The HR pathway catalyzes DSB repair by identifying of a stretch of homologous DNA and by replicating from this homologous DNA template, while NHEJ repairs DSBs by processing and re-ligating the DSB ends (Thompson, et al., 2001; Lieber, et al., 2004). Like HR, the canonical version of NHEJ is thought to repair DNA with high fidelity (Arlt, et al., 2012; Guirouilh-Barbat, et al., 2004). However, some DSBs can undergo extensive degradation prior to re-ligation using processes termed microhomology-mediated end joining and single-strand annealing, both of which create mutagenic deletions (Guirouilh-Barbat, et al., 2004; Bennardo, et al., 2008). Similarly, mutations can arise if replication-disrupting lesions are not properly repaired prior to DNA replication, in which case these lesions may prompt homology-mediated polymerase template switching (Malkova, et al., 2012).

The efficiencies of these repair processes have important implications for carcinogenesis and malignant tumor progression. Like HR, the canonical version of NHEJ is thought to repair DNA with high fidelity (Arlt, et al., 2012; Guirouilh-Barbet, et al., 2004). However, some DSBs can undergo extensive degradation prior to re-ligation using processes termed microhomology-mediated end joining and single-strand annealing, both of which create mutagenic deletions (Guirouilh-Barbat, et al., 2004; Bennardo, et al., 2008). Similarly, mutations can arise if replication-disrupting lesions are not properly repaired prior to DNA replication, in which case these lesions may prompt homology-mediated polymerase template switching (Malkova, et al., 2012).

The cellular efficiencies of these repair processes can directly impact tumor responsiveness during the treatment of cancer patients. The most striking examples are the hypersensitivities of HR-deficient tumors to PARP inhibitors (Bryant, et al., 2004; Farmer, et al., 2004; O'Shaughnessy, et al., 2011) or platinum-based chemotherapies (Edwards, et al., 2008; Sakai, et al., 2008). At present, however, available methods to measure HR proficiency from human tumor biopsy tissues are limited (Willers, et al., 2009; Birkelbach, et al., 2013). Methods for measuring NHEJ from clinical specimens are also limited. Some studies have measured the rate of DSB rejoining in tumors (e.g. H2AX phosphorylation kinetics), and rapid DSB rejoining may predict resistance of human tumors to radiotherapy and some chemotherapy drugs (reviewed in Redon, et al., 2012). However, a single method that could successfully predict the relative efficiencies of both HR and NHEJ is needed.

### SUMMARY OF THE INVENTION

In some embodiments, there are provided compositions and methods concerning methods for predicting efficacy of a DNA damaging agent for treating cancer, methods for evaluating treatment with a DNA damaging agent in a cancer patient, methods for treating a human cancer patient with a DNA damaging agent, methods for prognosing a cancer patient, and/or methods for using an algorithm to treat a cancer patient with a DNA damaging agent.

Such methods and compositions may involve methods comprising measuring the level of expression of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or 32, genes (or any range derivable therein) from a biological sample from the patient: RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3. In certain embodiments, the genes to be measured may include, include at least, or at most 1, 2, 3, 4, 5, 6, 7, 8, 9, 10, 11, or 12 genes (or any range derivable therein) chosen from among PARI, RAD51, BLM, RIF1, Ku80, BRCA1, RAD51AP1, AD54B, Plk1, BRCA2, RAD51C, and PALB2. In other embodiments, the genes to be measured may exclude secondary regulators of damage response such as TP53, PTEN, and cell cycle checkpoint genes or one or more genes of CtIP, RAD51B, DNA-PKcs, PIAS4, XRCC3, XRCC2, RAD52, XRCC4, Artemis, RAD51D, WRN, RAD54L, HELQ, MDC1, LIG, PIAS1, Fbh1, RNF8, RNF4, or TP53BP1. In certain embodiments, the expression level may be measured by measuring the transcription factor that up-regulates the DNA repair genes, such as MYC or STAT3. In particular embodiments, expression levels of RIF1, PARI, RAD51, and Ku80 are measured. In certain embodiments, those four genes of RIF1, PARI, RAD51, and Ku80 are the only genes whose expression is measured.

In further embodiments, the genes to be measured may be genes directly relevant to replication stress and/or the DSB repair pathway, particularly genes involved in the cellular preference toward homologous recombination (HR) versus non-homologous end joining (NHEJ). The genes may include one or more NHEJ genes, including NHEJ genes involved in binding of DNA ends, such as Ku70 or Ku80, or ligation of DNA ends, such as Artemis, DNA-pk, or XRCC4/Ligase IV. The genes may include one or more HR gens, such as genes that encode a mediator of RAD51 assembly, like Palb2, BRCA2, RAD52, XRCC3/RAD51C, XRCC2/RAD51B/RAD51C/RAD51D, or RAD51AP1, or a gene that encodes a helicase or translocase, like PARI, RAD54L, RAD54B, Fbh1, WRN, or HELQ. In further embodiments, the genes may include or exclude one or more genes involved in damage sensing, such as one or more genes of RPA, ATRIP, ATR, Mre11/Rad50/NBS1 or ATM.

Expression may be measured of gene transcripts or of polypeptides. Expression of gene transcripts can be evaluated using any number of assays, including but not limited to assays involving hybridization and/or amplification, such as a reverse-transcriptase polymerase chain reaction (RT-PCR), real-time PCR or qPCR, microarray hybridization, RNA sequencing, etc. Protein-based expression assays are also possible, such as with one or more antibodies specific to the polypeptide. Methods that may be employed include, but are not limited to, those discussed in US Patent Publication 20100216131, 20100210522, 20100167939, 20100159445, and 20100143247, all of which are hereby incorporated by reference.

In certain embodiments, the expression of any of these genes is overexpressed compared to a reference or control sample. In certain embodiments the reference or control reflects the level of one or more non-responders or poor responders or the level of a group of patients that may be either non-responders or poor responders or random responders. It is contemplated that in some embodiments, the highest levels of expression correspond to the greatest chance of efficacy. In some embodiments, the patient has a level of expression that places him/her in the top quarter or top half of responders as far as success of response. In other embodiments, the patient has a level of expression that places him/her in the top 10, 20, 30, 40, 50 percentile as compared to a control or reference level. In certain embodiments, the control or reference sample is the level for responders for that cancer therapy. In some embodiments, a level of expression of a particular gene may be compared to be both responders and non-responders.

In certain embodiments, methods comprise determining a response score that predicts the patient's resistance to a DNA-damaging chemotherapy and/or the patient's sensitivity to a DNA-damaging radiation therapy. The response score may be based on expression levels of the genes measured compared to control expression levels. The response score may be calculated based on the sum of the expression level of the genes selected from any genes mentioned herein. The response score may be a log transformation of the expression level and may also times -1 to generate a score less than 0. The response score may be a recombination proficiency score (RPS). The response score may be determined by a computer using an algorithm.

The DNA-damaging chemotherapy or DNA damaging agent may be a platinum-based compound, DNA cross-linker, a topoisomerase inhibitor, or a PARP inhibitor. In certain embodiments, the platinum-based compound may be cisplatin or carboplatin. In further embodiments, the topoisomerase inhibitor may be irinotecan or topotecan. In still further embodiments, a PARP inhibitor may be used as selected from the group consisting of a tetracycline compound, 4-hydroxyquinazoline and a derivative thereof, and a carboxamino-benzimidazole and a derivative thereof.

In some embodiments, methods are performed in vitro on a biological sample from the patient. The sample comprises cancer cells in some embodiments.

In further embodiments, the patient may be treated with the DNA damaging agent within or after 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 days, 1, 2, 3, 4, 5, 6, 7, 8, 9, 10, 11, 12, 13, 14, 15, 16, 17, 18, 19, 20 weeks, 1, 2, 3, 4, 5, 6, 7, 8, 9, 10, 11, 12, 13, 14, 15, 16, 17, 18, 19, 20 months (or any range or value derivable therein) after the patient has been determined to be a patient with predicted sensitivity to treatment with the DNA damaging agent. The patient may have previously undergone surgery as cancer treatment. The patient may be determined to be a patient with predicted sensitivity to treatment with the DNA damaging agent using an algorithm or may be predicted to be more likely to respond to a DNA-damaging chemotherapy than not. The response to a therapy may be defined as a reduction in tumor size by at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 50, 60, 70, 80, 99 or 100 % (or any range or value derivable therein) after a first or full course of treatment

The cancer involved may be basal cell carcinoma, biliary tract cancer; bladder cancer; bone cancer; brain and CNS cancer; breast cancer; cervical cancer; choriocarcinoma; colon and rectum cancer; connective tissue cancer; cancer of the digestive system; endometrial cancer; esophageal cancer; eye cancer; cancer of the head and neck; gastric cancer; intra-epithelial neoplasm; kidney cancer; larynx cancer; leukemia; liver cancer; lung cancer, small cell lung cancer, non-small cell lung cancer; lymphoma including Hodgkin's and non-Hodgkin's lymphoma; melanoma; myeloma; neuroblastoma; oral cavity cancer, lip cancer, tongue cancer, mouth cancer, pharynx cancer; ovarian cancer; pancreatic cancer; prostate cancer; retinoblastoma; rhabdomyosarcoma; rectal cancer; renal cancer; cancer of the respiratory system; sarcoma; skin cancer; stomach cancer; testicular cancer; thyroid cancer; uterine cancer; cancer of the urinary system, sarcoma, or metastatic cancer.

In further embodiments, methods may be provided for treating a human patient with cancer comprising treating the patient with a DNA damaging agent after the patient has been determined to be a patient with predicted sensitivity to treatment with the DNA damaging agent, wherein the determination is made based on measuring the level of expression of two or more of the following human genes, from a biological sample from the patient, RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3.

In still further embodiments, methods may be provided for using an algorithm to predict therapeutic efficacy of a DNA damaging agent on a cancer patient comprising measuring the level of expression of at least two of the following genes from a biological sample from the patient: PARI, BLM, RAD51, RIF1, BRCA1, Ku80, RAD51AP1, RAD54B, Plk1, BRCA2, RAD51C, PALB2, MYC, or STAT3, and calculating a response score that predicts the therapeutic efficacy of a DNA damaging agent on the cancer patient based on the level of expression.

In certain embodiments, methods may be provided for evaluating cancer treatment with a DNA damaging agent on a human cancer patient comprising measuring the level of expression of at least one human gene involved in the repair of double-stranded DNA breaks from a biological sample from the patient; comparing the level of expression to a reference or control level of expression of that gene; and, determining whether the patient is likely to have a positive response to the DNA damaging agent.

In further embodiments, methods may be provided for treating a human patient with cancer comprising measuring, in a tumor cell or tissue from the patient, the level of expression of two or more of genes selected from the group of RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, and STAT3. Methods may further comprise treating the patient with a DNA damaging agent after the patient has been determined to be a patient with predicted sensitivity to treatment with the DNA damaging agent. In other embodiments, there may be provided a method of treating a cancer patient comprising measuring, in a tumor cell or tissue from the patient, the level of expression of RIF1, PARI, RAD51 and Ku80.

The methods may further provide calculating a recombination proficiency (RPS) score from the measured level of expression. The methods may also comprise comparing the calculated RPS score with a reference RPS score. In further embodiments, the methods may comprise treating the patient with a DNA damaging agent if the calculated RPS score is lower than the reference RPS score.

In further embodiments, there may be provided a method of predict therapeutic efficacy of a treatment regimen comprising radiation, platinum-based compound, DNA cross-linker, a topoisomerase inhibitor, and/or a PARP inhibitor, said method comprising: measuring, in a tumor cell or tissue from a cancer patient, the expression level of two or more genes chosen from the group of PARI, BLM, RAD51, Rif1, BRCA1, Ku80, RAD51AP1, RAD54B, Plk1, BRCA2, RAD51C, PALB2, MYC, and STAT3; calculating a recombination proficiency (RPS) score using the measured gene expression levels; and comparing the calculated RPS score to a reference RPS score, wherein a calculated RPS score lower than the reference RPS score would indicate an increased likelihood of response by the patient to said treatment regimen.

The measuring step may comprise qPCR, RNA sequencing, microarray analysis, or any methods known in the art. In certain embodiments, the DNA damaging agent is radiation, platinum-based compound, DNA cross-linker, a topoisomerase inhibitor, or a PARP inhibitor. In further embodiments, the predicted response to radiation is the opposite to the predicted response to a DNA damaging chemotherapy.

In further embodiments, there may be provided kits comprising oligonucleotides, such as primers or probes, that bind or are capable of hybridizing, respectively, to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 (or any range derivable therein) genes or transcripts thereof selected from the group consisting of: RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3. In certain embodiments, the genes may be 1, 2, 3, 4, 5, 6, 7, 8, 9, 10, 11, or 12 genes of PARI, RAD51, BLM, RIF1, Ku80, BRCA1, RAD51AP1, AD54B, Plk1, BRCA2, RAD51C, and PALB2.

In other embodiments, the genes may exclude secondary regulators of damage response such as TP53, PTEN, and cell cycle checkpoint genes or one or more genes of CtIP, RAD51B, DNA-PKcs, PIAS4, XRCC3, XRCC2, RAD52, XRCC4, Artemis, RAD51D, WRN, RAD54L, HELQ, MDC1, LIG, PIAS1, Fbh1, RNF8, RNF4, or TP53BP1. In particular embodiments, the kit comprise oligonucleotides that bind or are capable of hybridizing, respectively, to two, three, four, five or six genes chosen from the group of RIF1, PARI, RAD51, Ku80, MYC and STAT3, or to all four genes of RIF1, PARI, RAD51, and Ku80. In particular embodiments, the kit includes one or more oligonucleotides capable of hybridizing to a RIF1 gene sequence, one or more oligonucleotides capable of hybridizing to a PARI gene sequence, one or more oligonucleotides capable of hybridizing to a RAD51 gene sequence, and one or more oligonucleotides capable of hybridizing to a Ku80 gene sequence.

Particularly, the oligonucleotides are 20 to 500 nucleotides long; in some embodiments they are 20 to 200 nucleotides in length. Each oligonucleotide may be a probe or primer that is labeled or unlabeled, and can hybridize under stringent hybridization conditions to an mRNA or cDNA encoded by one of the genes.

In further embodiments, the kids comprise labelled oligonucleotides, such as primers or probes. These labelled oligonucleotides are not naturally occuring and are markedly different from naturally occuring nucleotides in structure at least because they have non-nucleotide portions linked to nucleotides in a non-natural way.

The kits may be used in detecting gene expression in cells or tissue from a patient, particularly tumor tissue from a cancer patient. For example, in real-time TaqMan PCR, oligonucleotides may be used as PCR primers, and/or TaqMan probe. In sequencing, the oligonucleotides may be PCR primers and/or sequencing primers. In next-generation sequencing, the oligonucleotides may be used as capturing probes to capature targeted genes or mRNAs or cDNAs. In microarray-based gene expression profiling, the oligonucleotides may be probes attached to a solid support forming a hybridization chip.

Thus, the kits may additional include one or more reagents useful for PCR reactions, sequencing reactions, and/or hybridization reactions, such as Taq polymerase, reaction buffers, dNTPs, etc.

Other embodiments are set forth in the claims and in the disclosure.

Throughout this application, the term "about" is used to indicate that a value includes the inherent variation of error for the measurement or quantitation method.

The use of the word "a" or "an" when used in conjunction with the term "comprising" may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of" any of the ingredients or steps disclosed throughout the specification. Compositions and methods "consisting essentially of" any of the ingredients or steps disclosed limits the scope of the claim to the specified materials or steps which do not materially affect the basic and novel characteristic of the claimed invention.

It is contemplated that any embodiment discussed in this specification can be implemented with respect to any method or composition of the invention, and vice versa. Furthermore, compositions of the invention can be used to achieve methods of the invention.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description. Note that simply because a particular compound is ascribed to one particular generic formula doesn't mean that it cannot also belong to another generic formula.

Moreover, the invention relates to the following embodiments:
1. A method for treating a human cancer patient comprising administering a DNA damaging agent after the patient has been determined to have predicted sensitivity to treatment with the DNA damaging agent, wherein the determination is made based on a recombination proficiency (RPS) score generated from the measured level of expression of RIF1, PARI, RAD51 or Ku80 in a biological sample of the patient.
2. The method of item 1, wherein the DNA damaging agent is radiation, a platinum-based compound, DNA cross-linker, a topoisomerase inhibitor, or a PARP inhibitor.
3. A method for treating a human cancer patient comprising administering a DNA damaging agent after the patient has been determined to have predicted sensitivity to treatment with the DNA damaging agent, wherein the determination is made based on measuring the level of expression of one or more human genes involved in the repair of double-stranded DNA breaks from a biological sample from the patient.
4. The method of item 3, wherein the method comprises determining a response score that predicts the patient's resistance to a DNA-damaging chemotherapy and/or the patient's sensitivity to a DNA-damaging radiation therapy.
5. The method of item 4, wherein the response score predicts the patient's resistance to a DNA-damaging chemotherapy.
6. The method of item 4, wherein the response score predicts the patient's sensitivity to a DNA-damaging radiation therapy.
7. The method of item 4, wherein the response score is determined by a computer using an algorithm.
8. The method of any of items 3-7, wherein the one or more human genes comprise three or more of RIF1, PARI, RAD51 or Ku80.
9. The method of any of items 3-8, wherein the one or more human genes comprise all genes of RIF1, PARI, RAD51 and Ku80.
10. The method of any of items 3-9, wherein the one or more human genes comprise one or more genes of RIF1, PARI, RAD51, Ku80, MYC, or STAT3.
11. The method of any of items 3-10, wherein the one or more human genes comprise a non-homologous end joining (NHEJ) gene.
12. The method of item 11, wherein the NHEJ gene is Ku70, Ku80, artemis, DNA-pk, or XRCC4/Ligase IV.
13. The method of any of items 3-12, wherein the one or more human genes comprise a homologous recombination (HR) gene.
14. The method of item 13, wherein the HR gene encodes a mediator of RAD51 assembly.
15. The method of item 14, wherein the mediator of RAD51 assembly is Palb2, BRCA2, RAD52, XRCC3/RAD51C, XRCC2/RAD51B/RAD51D, or RAD51AP1.
16. The method of item 13, wherein the HR gene encodes a helicase or translocase.
17. The method of item 16, wherein the helicase or translocase is BLM, PAR, RAD54L, RAD54B, Fbh1, or WRN.
18. The method of item 13, wherein the HR gene is Rad 51, Palb2, BRCA2, RAD52, XRCC3/RAD51C, XRCC2/RAD51B/RAD51D, RAD51AP1, BLM, PAR, RAD54L, RAD54B, Fbh1, or WRN.
19. The method of any of items 3-18, wherein the one or more human genes comprise a gene involved in damage sensing.
20. The method of item 19, wherein the gene involved in damage sensing is RPA, ATRIP, ATR, Mre11/Rad50/NBS1 or ATM.
21. The method of any of items 3-20, wherein the one or more human genes comprise a gene involved in decision point or DNA end resection.
22. The method of any of items 3-21, wherein the one or more human genes comprise one or more genes of MDC1, BRCA1, 53BP1, CtIP, or Rif1.
23. The method of any of items 3-22, wherein the one or more human genes comprise one or more genes of RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3.
24. The method of any of items 3-23, wherein the level of expression of at least two genes involved in the repair of double-stranded DNA breaks is measured.
25. The method of any of items 3-24, wherein the DNA damaging agent is radiation.
26. The method of any of items 3-25, wherein the DNA damaging agent is a platinum-based compound, DNA cross-linker, a topoisomerase inhibitor, or a PARP inhibitor.
27. The method of item 26, wherein the platinum-based compound is cisplatin or carboplatin.
28. The method of item 26, wherein the topoisomerase inhibitor is irinotecan or topotecan.
29. The method of any of items 3-28, wherein the patient is treated with the DNA damaging agent within 1 month after the patient has been determined to be a patient with predicted sensitivity to treatment with the DNA damaging agent.
30. The method of any of items 3-29, wherein the patient previously underwent surgery as cancer treatment.
31. The method of any of items 3-30, wherein the one or more human genes are overexpressed compared to the level of expression in a control.
32. The method of any of items 3-31, wherein the patient is determined to be a patient with predicted sensitivity to treatment with the DNA damaging agent using an algorithm.
33. The method of item 32, wherein the algorithm is based on a response score of the measured expression levels of the genes compared to control expression levels.
34. The method of any of items 3-33, wherein the cancer is basal cell carcinoma, biliary tract cancer; bladder cancer; bone cancer; brain and CNS cancer; breast cancer; cervical cancer; choriocarcinoma; colon and rectum cancer; connective tissue cancer; cancer of the digestive system; endometrial cancer; esophageal cancer; eye cancer; cancer of the head and neck; gastric cancer; intra-epithelial neoplasm; kidney cancer; larynx cancer; leukemia; liver cancer; lung cancer, small cell lung cancer, non-small cell lung cancer; lymphoma including Hodgkin's and non-Hodgkin's lymphoma; melanoma; myeloma; neuroblastoma; oral cavity cancer, lip cancer, tongue cancer, mouth cancer, pharynx cancer; ovarian cancer; pancreatic cancer; prostate cancer; retinoblastoma; rhabdomyosarcoma; rectal cancer; renal cancer; cancer of the respiratory system; sarcoma; skin cancer; stomach cancer; testicular cancer; thyroid cancer; uterine cancer; cancer of the urinary system, sarcoma, or metastatic cancer.
35. A method for treating a human patient with cancer comprising treating the patient with a DNA damaging agent after the patient has been determined to be a patient with predicted sensitivity to treatment with the DNA damaging agent, wherein the determination is made based on measuring the level of expression of two or more of the following human genes, from a biological sample from the patient, RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3.
36. The method of item 35, wherein the two or more human genes comprises PARI, BLM, RAD51, Rif1, BRCA1, Ku80, RAD51AP1, RAD54B, Plk1, BRCA2, RAD51C, PALB2, MYC, or STAT3.
37. The method of item 35, wherein the determination is made based on the level of expression of at least RIF1, PARI, RAD51, or Ku80.
38. A method of using an algorithm to predict therapeutic efficacy of a DNA damaging agent on a cancer patient comprising measuring the level of expression of at least two of the following genes from a biological sample from the patient: PARI, BLM, RAD51, Rif1, BRCA1, Ku80, RAD51AP1, RAD54B, Plk1, BRCA2, RAD51C, PALB2, MYC, or STAT3, and calculating a response score that predicts the therapeutic efficacy of a DNA damaging agent on the cancer patient based on the level of expression.
39. The method of item 38, wherein the level of expression of at least RIF1, PARI, RAD51, and Ku80 are measured.
40. A method for evaluating cancer treatment with a DNA damaging agent on a human cancer patient comprising measuring the level of expression of at least one human gene involved in the repair of double-stranded DNA breaks from a biological sample from the patient; comparing the level of expression to a reference or control level of expression of that gene; and, determining whether the patient is likely to have a positive response to the DNA damaging agent.
41. The method of item 40, wherein the one or more genes are RIF1, PARI, RAD51, Ku80, MYC, or STAT3.
42. The method of item 40, wherein the gene involved in the repair of double-stranded DNA breaks is involved in non-homologous end joining (NHEJ).
43. The method of item 40, wherein the NHEJ gene comprises a gene of Ku70, Ku80, artemis, DNA-pk, or XRCC4/Ligase IV.
44. The method of item 40, wherein the gene involved in the repair of double-stranded DNA breaks is involved in homologous recombination (HR).
45. The method of item 44, wherein the HR gene encodes a mediator of RAD51 assembly.
46. The method of item 45, wherein the mediator of RAD51 assembly is Palb2, BRCA2, RAD52, XRCC3/RAD51C, XRCC2/RAD51B/RAD51D, or RAD51AP1.
47. The method of item 44, wherein the HR gene encodes a helicase or translocase.
48. The method of item 47, wherein the helicase or translocase is BLM, PAR, RAD54L, RAD54B, Fbh1, or WRN.
49. The method of item 44, wherein the HR gene comprises a gene of Rad 51, Palb2, BRCA2, RAD52, XRCC3/RAD51C, XRCC2/RAD51B/RAD51D, RAD51AP1, BLM, PAR, RAD54L, RAD54B, Fbh1, or WRN.
50. The method of item 40, wherein the at least one human gene comprises a gene involved in damage sensing.
51. The method of item 50, wherein the gene involved in damage sensing is RPA, ATRIP, ATR, Mre11/Rad50/NBS1 or ATM.
52. The method of item 40, wherein the at least one human gene comprises a gene involved in decision point or DNA end resection.
53. The method of item 52, wherein the gene involved in decision point or DNA end resection comprises a gene of MDC1, BRCA1, 53BP1, CtIP, or Rif1.
54. The method of item 40, wherein the at least one human gene comprises a gene of RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, or STAT3.
55. The method of any of items 40-54, wherein the level of expression of at least two genes involved in the repair of double-stranded DNA breaks is determined.
56. The method of any of items 40-55, wherein the DNA damaging agent is radiation.
57. The method of any of items 40-55, wherein the DNA damaging agent is a platinum-based compound, DNA cross-linker, a topoisomerase inhibitor, or a PARP inhibitor.
58. The method of item 57, wherein the topoisomerase inhibitor is irinotecan or topotecan
59. The method of item 57, wherein the platinum-based compound is cisplatin or carboplatin.
60. The method of any of items 40-59, further comprising treating the patient with the DNA damaging agent within 1 month after the patient has been determined to be a patient with predicted sensitivity to treatment with the DNA damaging agent.
61. The method of any of items 40-60, wherein the patient previously underwent surgery as cancer treatment.
62. The method of any of items 40-61, wherein the one or more genes is overexpressed compared to the level of expression in a control.
63. The method of any of items 40-62, further comprising using an algorithm to predict sensitivity to treatment with the DNA damaging agent using an algorithm.
64. The method of any of items 40-63, wherein the cancer is basal cell carcinoma, biliary tract cancer; bladder cancer; bone cancer; brain and CNS cancer; breast cancer; cervical cancer; choriocarcinoma; colon and rectum cancer; connective tissue cancer; cancer of the digestive system; endometrial cancer; esophageal cancer; eye cancer; cancer of the head and neck; gastric cancer; intra-epithelial neoplasm; kidney cancer; larynx cancer; leukemia; liver cancer; lung cancer, small cell lung cancer, non-small cell lung cancer; lymphoma including Hodgkin's and non-Hodgkin's lymphoma; melanoma; myeloma; neuroblastoma; oral cavity cancer, lip cancer, tongue cancer, mouth cancer, pharynx cancer; ovarian cancer; pancreatic cancer; prostate cancer; retinoblastoma; rhabdomyosarcoma; rectal cancer; renal cancer; cancer of the respiratory system; sarcoma; skin cancer; stomach cancer; testicular cancer; thyroid cancer; uterine cancer; cancer of the urinary system, sarcoma, or metastatic cancer.
65. The method of any of items 40-64, wherein the level of expression is determined using a hybridization or/or amplification assay.
66. A method for treating a human patient with cancer comprising:
   measuring, in a tumor cell or tissue from the patient, the level of expression of two or more of genes selected from the group of RPA, ATRIP, ATR, Mre11/Rad50/NBS1, ATM, MDC1, BRCA1, 53BP1, CtIP, RIF1, Ku70, Ku80, artemis, DNA-pk, XRCC4/Ligase IV, RAD51, Palb2, BRCA2, RAD52, XRCC3/RAD51C, XRCC2/RAD51B/RAD51D, RAD51AP1, BLM, PAR, RAD54L, RAD54B, Fbh1, WRN, PARI, HELQ, MYC, and STAT3; and
   treating the patient with a DNA damaging agent after the patient has been determined to be a patient with predicted sensitivity to treatment with the DNA damaging agent.
67. A method of treating a cancer patient comprising:
   measuring, in a tumor cell or tissue from the patient, the level of expression of RIF1, PARI, RAD51 and Ku80;
   calculating a recombination proficiency (RPS) score from the measured level of expression;
   comparing the calculated RPS score with a reference RPS score; and
   treating the patient with a DNA damaging agent if the calculated RPS score is lower than the reference RPS score.
68. The method of item 67, wherein the measuring step comprises qPCR, RNA sequencing, or microarray analysis.
69. The method of item 67, wherein the DNA damaging agent is radiation, platinum-based compound, DNA cross-linker, a topoisomerase inhibitor, or a PARP inhibitor.
70. A method of predict therapeutic efficacy of a treatment regimen comprising radiation, platinum-based compound, DNA cross-linker, a topoisomerase inhibitor, and/or a PARP inhibitor, said method comprising:
   measuring, in a tumor cell or tissue from a cancer patient, the expression level of two or more genes chosen from the group of PARI, BLM, RAD51, Rif1, BRCA1, Ku80, RAD51AP1, RAD54B, Plk1, BRCA2, RAD51C, PALB2, MYC, and STAT3;
   calculating a recombination proficiency (RPS) score using the measured gene expression levels; and
   comparing the calculated RPS score to a reference RPS score, wherein a calculated RPS score lower than the reference RPS score would indicate an increased likelihood of response by the patient to said treatment regimen.
71. The method of item 70, wherein the level of expression of at least RIF1, PARI, RAD51, and Ku80 are measured.
72. The method of item 70 or 71, wherein the measuring step comprises qPCR, RNA sequencing, or microarray analysis.
73. A method of providing a recombination proficiency score (RPS) in a cell or tissue from a patient, comprising:
   measuring the expression level of the genes RIF1, PARI, RAD51, and Ku80 in the cell or tissue; and
   providing a RPS as the sum of these four expression levels multiplied times -1, using the log2-transformed normalized mRNA values of each gene.
74. The method of item 73, wherein the measuring step comprises qPCR, RNA sequencing, or microarray analysis.
75. A kit comprising oligonucleotides capable of hybridizing, respectively, to at least two genes chosen from the group of PARI, BLM, RAD51, Rif1, BRCA1, Ku80, RAD51AP1, RAD54B, Plk1, BRCA2, RAD51C, PALB2, MYC, and STAT3.
76. A kit comprising oligonucleotides capable of hybridizing, respectively, to at least two genes chosen from the group consisting of PARI, Rifl, Ku80, RAD51, BLM, BRCA1, RAD51AP1 and RAD54B.
77. A kit comprising oligonucleotides capable of hybridizing, respectively, to at least two genes chosen from the group consisting of PARI, Rif1, Ku80 and RAD51.

### BRIEF DESCRIPTION OF THE DRAWINGS

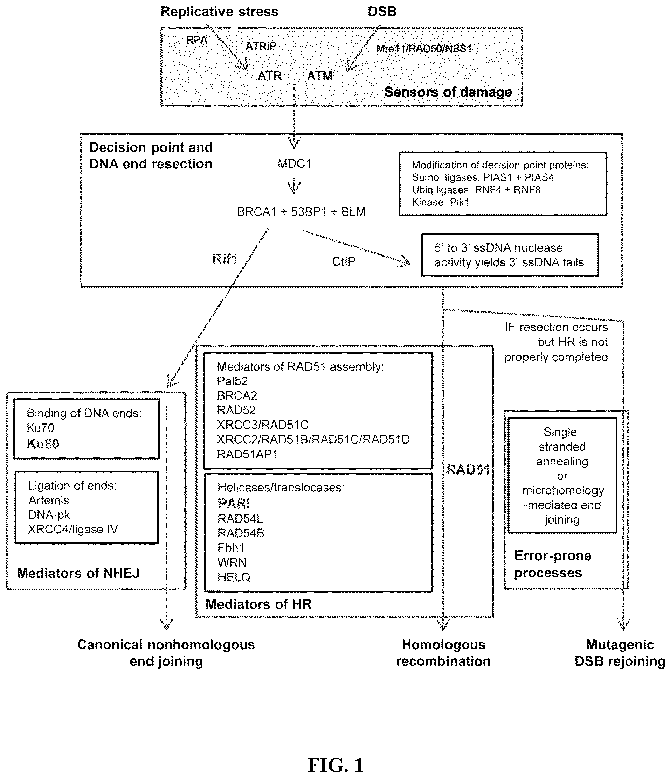

**FIG. 1** **Pathways and genes involved in repair of double-strand DNA breaks (DSBs) and the tolerance of replication stress.** Shown is a simplified overview of the mechanistic steps and genes involved in DNA repair, with an emphasis on those that facilitate homologous recombination and non-homologous end joining. All of the displayed genes were considered candidates for the Recombination Proficiency Score (RPS) system, except those within the blue box. The four genes whose expression levels were ultimately chosen to comprise the RPS are displayed in red.

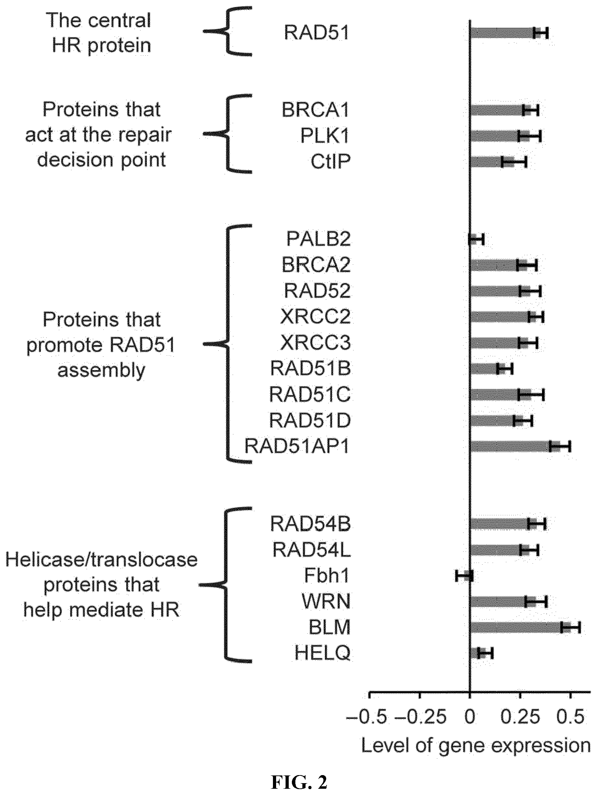

**FIG. 2** **Cell lines with low RPS overexpress a wide array of HR-related genes.** Mean mRNA levels are shown for the CCLE cell lines with low RPS scores. These mRNA levels were mined from the CCLE database, and displayed values represent log2 transformed mRNA measurements of each gene normalized to the median mRNA among the starting 634 carcinoma cell lines. Therefore an expression level of zero indicates a median expression level, and any positive value indicates overexpression. For example, a value of +0.25 indicates a 19% increase in expression above the median. Error bars denote standard error.

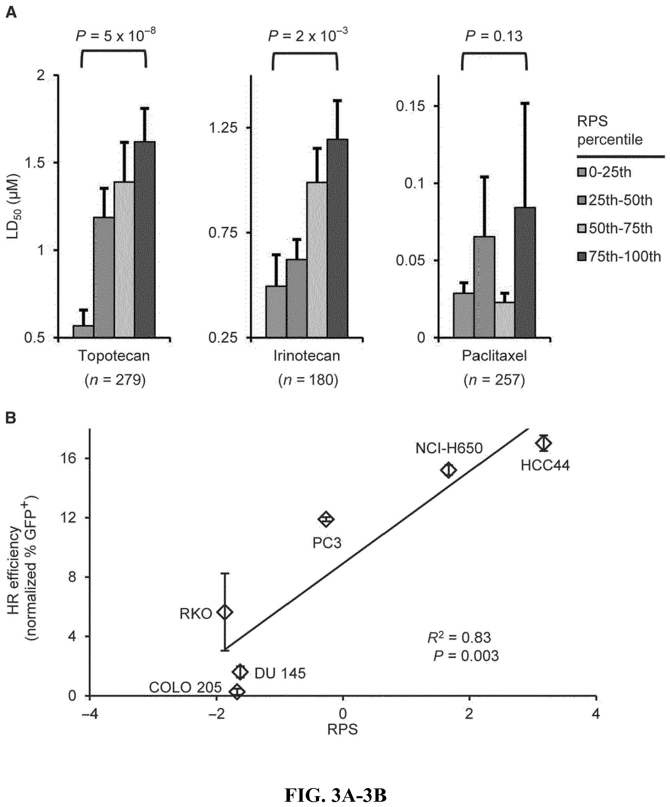

**FIGS. 3A-3B** **RPS correlates with sensitivity to different classes of treatment and HR deficiency in cell lines. A)** CCLE carcinoma cell lines were binned into quartiles, based on RPS. Sensitivity data were mined from the CCLE database and plotted for different oncologic therapies, and differences between the highest and lowest quartiles were determined by Student's T test. **B)** HR repair efficiency correlates with RPS. Six representative cell lines were co-transfected with an HR reporter-containing plasmid (pDR-GFP) plus an I-Sce I expressing plasmid (pCβASce) or an empty vector control plasmid (pCAG), and were subjected to FACS analysis 48 hours later. Reported HR efficiency represents the percent GFP+ cells with pDR-GFP + pCβASce, normalized to background (pDR-GFP + pCAG).

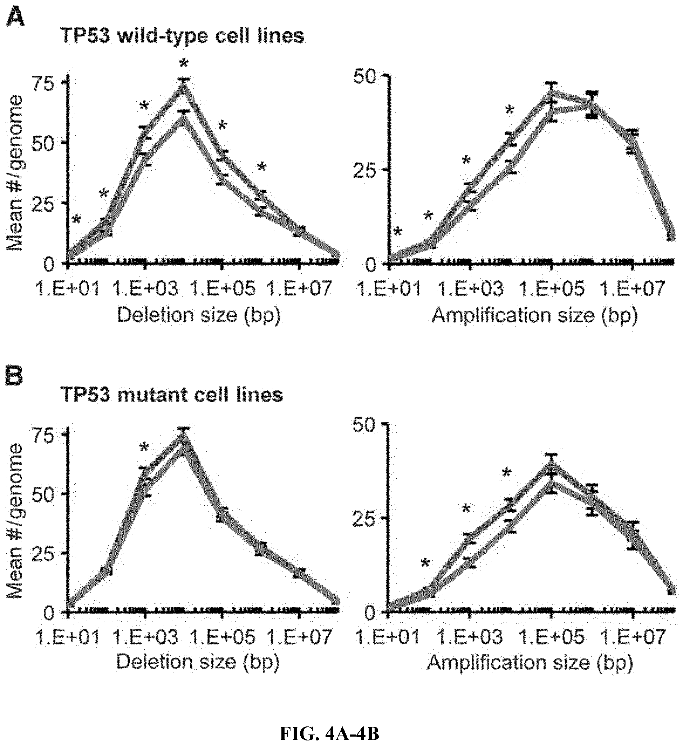

**FIGS. 4A-4B** **CCLE carcinoma cell lines with low RPS have elevated genomic instability.** SNP array-based DNA copy number variations (CNVs) were mined from the CCLE database. DNA deletions (left) and amplifications (right) were binned by size, wherein bins represent 10-fold increments in mutation size. High and low RPS groups were defined as the top and bottom quartiles, respectively. Size-based distributions of CNVs are shown for **A)** TP53 WT cells and **B)** TP53 mutant cells. Error bars denote standard error. Asterisks denote significant differences, based on Student's T test.

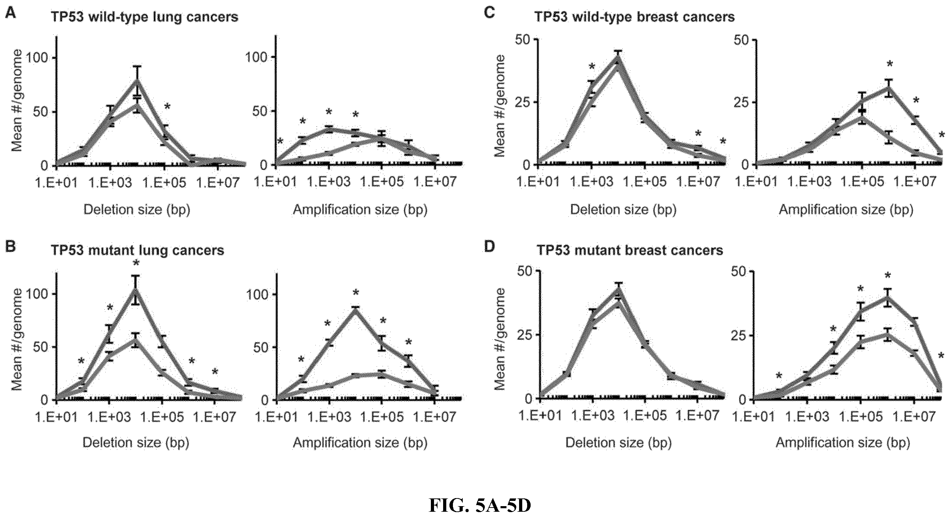

**FIGS. 5A-5D** **Low RPS is associated with genomic instability in human tumors.** SNP array-based DNA copy number variations (CNVs) were mined from the Cancer Genome Atlas. DNA deletions (left) and amplifications (right) were binned by size, wherein bins represent 10-fold increments in size. High and low RPS groups were defined as the top and bottom quartiles, respectively. Size-based distributions of CNVs are shown for **A)** TP53 WT NSCLC tumors, **B)** TP53 mutant NSCLC tumors, **C)** TP53 WT breast tumors, **D)** TP53 mutant breast tumors. Error bars denote standard error. Asterisks denote significant differences, based on Student's T test.

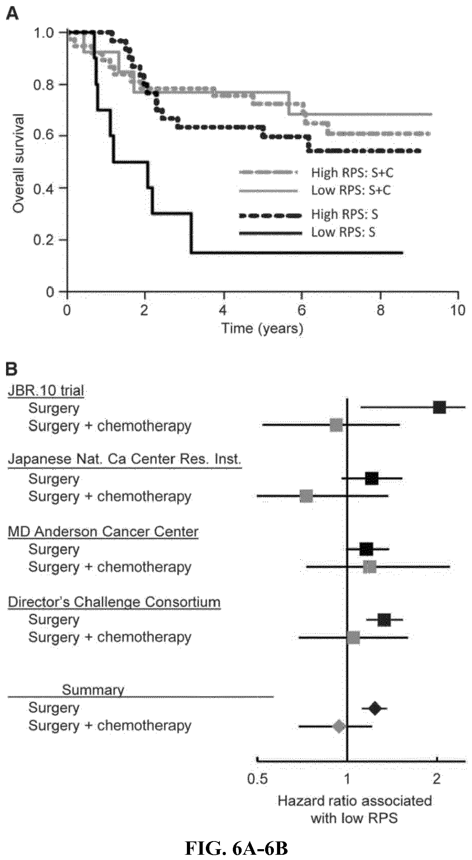

**FIGS. 6A-6B** **RPS is prognostic and correlates with treatment sensitivity in clinical tumors. A)** Kaplan Meier survival curves are shown for NSCLC patients treated on the JBR.10 trial with either surgery alone (S) or surgery followed by chemotherapy (S+C). Low and high RPS groups were defined as the bottom 25^{th} percentile and the remaining upper 75^{th} percentile, respectively. **B)** Four clinical datasets of non-small cell lung cancer were analyzed for prognostic impact of RPS on survival, using multivariate analyses that controlled for overall stage. Points in the Forest plot represent treatment-specific hazard ratios of RPS (as a continuous variable). Boxes denote hazard ratio and diamonds denote modeled hazard ratio values that summarize the combined impact of all four datasets. Error bars denote 95% confidence intervals. Black= surgery alone, green= surgery + chemotherapy.

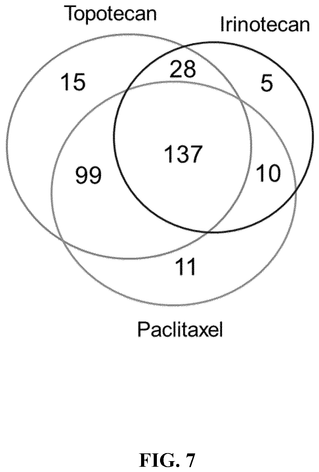

**FIG. 7** **Most of the drug sensitivity values mined from the CCLE database were generated in the same cell lines.** A Venn diagram displays the number of CCLE carcinoma cell lines for which treatment sensitivity data (topotecan, irinotecan, or paclitaxel) was available.

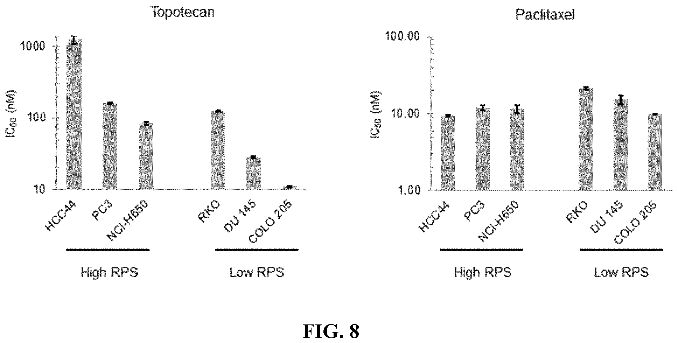

**FIG. 8** **A representative panel of cancer cell lines exhibits expected levels of drug resistance.** Cells were plated into 96-well tissue culture plates. The indicated for drugs were added for three days thereafter, and average survival from six replicates was measured using CellGlo reagent (Promega). Error bars represent the standard error.

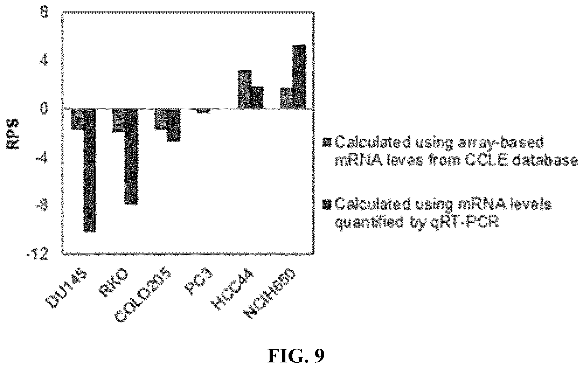

**FIG. 9** **Measurements of mRNA by real time qRT-PCR for six representative cell lines generated RPS values that were comparable to RPS values calculated from array-based mRNA levels reported in the CCLE database.** Cells were grown to 70% confluence and mRNA was isolated with TRIzol (Life Technologies) using the manufacturer's instructions. The resulting mRNA was quantified using the Qubit RNA BR assay (LifeTechnologies). An equal amount of RNA (1.5 µg) from each cell line was treated with DNAse-I (ThermoScientific), and cDNA was synthesized using Applied Biosystems High Capacity cDNA Reverse Transcription Kit (LifeTechnologies). PCR was performed with an Applied Biosystems 7900HT Sequence Detection System, using Applied Biosystems 2X Taqman Universal Master Mix II. These qRT-PCR derived mRNA levels were normalized to the levels in PC3 cells, log2 transformed, and summed to generate an RPS value for each cell line. The following Taqman Assay components were used in the PCR reaction: Ku80 (also known as XRCC5): Hs00897854_m1; RIF1: Hs00871714_m1; PARI (also known as PARPBP): Hs01550690_m1; RAD51: Hs00153418_m1.

### DETAILED DESCRIPTION OF THE INVENTION

Human tumors exhibit a wide range of malignant features and responsiveness to treatments that damage DNA. The inventors hypothesized that a component of this variability can be explained by differential efficiencies of DNA repair pathways. To study this further the inventors developed an analytic tool to indirectly quantify the efficiency of HR in individual cancers. This scoring system may be based on the expression of four DNA repair genes in a tumor cell or tissue: RIF1, PARI, RAD51, and Ku80. In certain examples, it was shown here that the Recombination Proficiency Score (RPS) correlates with sensitivity to specific classes of chemotherapy, associates with degree of genomic instability within tumor cells, and provides valuable information that is not available using existing diagnostic methods.

### I. Response Score

In certain embodiments, a response score may be determined based on the expression level of one or more genes involved in DNA repair pathways, such as replicative stress and the DSB repair pathways, or more particularly, the genes involved in cellular preference toward HR versus NHEJ. The Score may be expressed as a Recombination Proficiency Score (RPS).

In particular embodiments, the RPS score may be based on the expression of four DNA repair genes: RIF1, PARI, RAD51, and Ku80. It is shown herein that the RPS correctly predicts sensitivity to various classes of DNA-damaging treatment, correlates with degree of genomic instability within tumor cells, and provides valuable prognostic information that is not available using existing diagnostic methods. The RPS is a novel scoring system that quantifies the expression of four genes to predict DSB repair pathway preference. In particular, mRNA levels for relevant DNA repair genes in carcinoma cell lines were compared to topotecan sensitivity. This identified a gene expression scoring system termed the Recombinant Proficiency Score (RPS) that is in inverse relationship with the expression level of repair genes. Low RPS can identify tumors that harbor HR suppression and hypersensitivity to specific chemotherapeutic classes.

When faced with a DSB, the cell's decision of whether to utilize HR vs. NHEJ is influenced by the cell cycle stage. NHEJ is the dominant pathway for repairing DSBs during G0/G1 stages of the cell cycle, while HR occurs generally during S and G2. This regulation of repair is governed primarily by BRCA1 and 53BP1 proteins, which compete for occupancy at the DSB site (Chapman, et al., 2013). Stabilization of 53BP1 in cooperation with RIF1 leads to the exclusion of BRCA1 protein from the repair complex, and the DSB subsequently progresses to repair by NHEJ (Zimmermann, et al., 2013; Chapman, et al., 2013). If 53BP1 is excluded from the repair complex, then the DSB progresses to repair by HR. In this case, the DSB ends are processed into HR substrates, which involves 5' to 3' nuclease activity that generates 3' single-stranded DNA tails. This end processing is promoted by several proteins including CtIP, BRCA1, and the MRN (Mre11/RAD50/NBS1) complex. The nuclease activity is also specifically triggered by interactions between Mre11 and cyclin dependent kinase 2, thereby promoting the phosphorylation of CtIP preferentially in S/G2 cells (Buis, et al., 2012).

Given the wide biological diversity known to exist between different classes of human malignancies, the analysis was limited to cell lines derived only from carcinomas. Cellular resistance to the topoisomerase-I inhibiting drug topotecan was selected as a surrogate marker for HR proficiency. Topotecan is a derivative of camptothecin, and this class of drugs was selected because it disrupts replication forks and exerts toxicity preferentially in cells that harbor HR defects (Nitiss, et al., 1988; Arnaudeau, et al., 2001). Topotecan IC50 data were available for 279 of the 634 carcinoma cell lines.

To focus the inventors analysis on the primary cellular features that mediate specific phenotypes, the analysis was restricted to genes with direct relevance to replication stress and the DSB repair pathways. The analysis was further limited to 31 central proteins that participate in cellular preference toward HR vs. NHEJ, following the ataxia telangiectasia mutated (ATM) and/or ataxia telangiectasia and Rad3-related protein (ATR) activation steps of DNA damage response. Secondary regulators of damage response (like TP53, PTEN, and cell cycle checkpoint genes) were not considered as gene candidates for the scoring system, since they exert cellular influences that extend beyond the scope of replication stress and DSB repair. Pearson's correlation analyses demonstrated that 12 of the final list of 31 candidate genes had expression levels that significantly correlated (defined as p<0.05) with cellular sensitivity to topotecan. In all 12 cases, increasing gene expression levels directly correlated with increasing topotecan sensitivity.

In certain embodiments, the response score, such as a RPS (Recombinant Proficiency Score), can be calculated using techniques for measuring gene expression, including, but not limited to, NanoString and RT-PCR. In certain embodiments, the RPS score may be calculated from microarray. In further embodiments, when using microarray data (from which all of our current data have come), there are methods that may be used for normalizing the raw gene expression values. For example, the data may be normalized as such: Raw Affymetrix CEL files were converted to a single value for each probe set using Robust Multi-array Average (RMA) and normalized using quantile normalization. Either the original Affymetrix U133+2 CDF file or a redefined custom CDF file (ENTREZG - v15) was used for the summarization. Any methods known in the art may be used for microarray data normalization and pre-processing and calculation of RPS values may be feasible across various methods of normalization.

There may also be methods for calculating RPS with modalities other than microarray. Although most studies using RT-PCR generally normalize to some sort of housekeeper gene (like actin or a ribosomal RNA), the inventors actually found the raw data of gene expression of the RPS genes worked the best (i.e. agreed best with the microarray data, again Sean may want to elaborate). In a particular embodiment, a NanoString-based method may be used to measure the degraded forms of mRNA that are generally found in typical patient tumor biopsy material (formalin fixed paraffin-embedded tissue). For this, the NanoString company may provide probes and housekeeping genes to serve as normalization controls.

In certain embodiments, the information provided by RPS may be a continuous variable or not a continuous variable. This depends somewhat on the source of the material (i.e. tumor type). When plotting RPS-based data, the highest and lowest quartiles may be focused, because that makes the result more visually obvious and more statistically significant. However the effect may be continuous over the full range of RPS values. In fact, the Forest plot uses RPS as a continuous variable to calculate hazard ratios.

### II. Use of the Response Score

In some aspects, embodiments comprise treating a subject with a specific therapeutic agent or evaluating efficacy of treatment. Examples of therapeutic agents (anticancer agents) include, but are limited to, e.g., chemotherapeutic agents, growth inhibitory agents, cytotoxic agents, agents used in radiation therapy, anti-angiogenesis agents, apoptotic agents, anti-tubulin agents, and other-agents to treat cancer, such as anti-HER-2 antibodies, anti-CD20 antibodies, an epidermal growth factor receptor (EGFR) antagonist (e.g., a tyrosine kinase inhibitor), HER1/EGFR inhibitor (e.g., erlotinib (Tarceva™), platelet derived growth factor inhibitors (e.g., Gleevec™ (Imatinib Mesylate)), a COX-2 inhibitor (e.g., celecoxib), interferons, cytokines, antagonists (e.g., neutralizing antibodies).

In certain embodiments, the patient's sensitivity to a chemotherapeutic agent positively correlates with the expression level of the genes described herein. The chemotherapeutic agent may be a platinum-based compound, such as cisplatin, carboplatin, oxaliplatin, satraplatin, picoplatin, Nedaplatin, Triplatin, Lipoplatin, or a liposomal version of cisplatin.

In certain embodiments, the chemotherapeutic agent may be a DNA cross-linker. Alkylating agents such as 1, 3-bis(2-chloroethyl)-1-nitrosourea (BCNU, carmustine)) and nitrogen mustard which are used in chemotherapy can cross link with DNA at N7 position of guanine on the opposite strands forming interstrand crosslink. Cisplatin (cis-diamminedichloroplatinum(II)) and its derivatives forms DNA cross links as monoadduct, interstrand crosslink, intrastrand crosslink or DNA protein crosslink. Mostly it acts on the adjacent N-7 guanine forming 1, 2 intrastrand crosslink.

In further embodiments, the chemotherapeutic agent may be a topoisomerase inhibitor. Topoisomerase inhibitors are drugs that affect the activity of two enzymes: topoisomerase I and topoisomerase II. When the DNA double-strand helix is unwound, during DNA replication or transcription, for example, the adjacent unopened DNA winds tighter (supercoils), like opening the middle of a twisted rope. The stress caused by this effect is in part aided by the topoisomerase enzymes. They produce single- or double-strand breaks into DNA, reducing the tension in the DNA strand. This allows the normal unwinding of DNA to occur during replication or transcription. Inhibition of topoisomerase I or II interferes with both of these processes. Two topoisomerase I inhibitors, irinotecan and topotecan, are semi-synthetically derived from camptothecin, which is obtained from the Chinese ornamental tree Camptotheca acuminate. Drugs that target topoisomerase II can be divided into two groups. The topoisomerase II poisons cause increased levels enzymes bound to DNA. This prevents DNA replication and transcription, causes DNA strand breaks, and leads to programmed cell death (apoptosis). These agents include etoposide, doxorubicin, mitoxantrone and teniposide. The second group, catalytic inhibitors, are drugs that block the activity of topoisomerase II, and therefore prevent DNA synthesis and translation because the DNA cannot unwind properly. This group includes novobiocin, merbarone, and aclarubicin, which also have other significant mechanisms of action

In still further embodiments, the chemotherapeutic agent may be a PARP inhibitor. As used herein, "PARP inhibitor" (i.e., an inhibitor of poly ADP ribose polymerase) shall mean an agent that inhibits PARP more than it inhibits any other polymerase. In one embodiment, the PARP inhibitor inhibits PARP at least two-fold more than it inhibits any other polymerase. In another embodiment, the PARP inhibitor inhibits PARP at least 10-fold more than it inhibits any other polymerase. In a third embodiment, the PARP inhibitor inhibits PARP more than it inhibits any other enzyme. In one particular embodiment, the PARP inhibitor is olaparib, rucaparib, veliparib, CEP 9722, MK 4827, BMN-673, 3-aminobenzamide, a tetracycline compound, 4-hydroxyquinazoline and a derivative thereof, and a carboxamino-benzimidazole and a derivative thereof,

In some embodiments, the chemotherapeutic agent is any of (and in some embodiments selected from the group consisting of) alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegaI1 (see, e.g., Agnew, Chem. Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Ill.), and TAXOTERE® doxetaxel (Rhone-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin. Additional chemotherapeutic agents include the cytotoxic agents useful as antibody drug conjugates, such as maytansinoids (DM1, for example) and the auristatins MMAE and MMAF, for example.

The chemotherapeutic agents may be administered serially (within minutes, hours, or days of each other) or in parallel; they also may be administered to the patient in a pre-mixed single composition. It is contemplated that a therapy as disclosed herein may be used *in vitro* or *in vivo.* These processes may involve administering several agents at the same time or within a period of time wherein separate administration of the substances produces a desired therapeutic benefit. This may be achieved by contacting the cell, tissue, or organism with a composition, such as a pharmaceutically acceptable composition, that includes two or more agents, or by contacting the cell with two or more distinct compositions, wherein one composition includes one agent and the other includes another.

"Prognosis" refers to as a prediction of how a patient will progress, and whether there is a chance of recovery. "Cancer prognosis" generally refers to a forecast or prediction of the probable course or outcome of the cancer, with or without a treatment. As used herein, cancer prognosis includes the forecast or prediction of any one or more of the following: duration of survival of a patient susceptible to or diagnosed with a cancer, duration of recurrence-free survival, duration of progression free survival of a patient susceptible to or diagnosed with a cancer, response rate in a group of patients susceptible to or diagnosed with a cancer, duration of response in a patient or a group of patients susceptible to or diagnosed with a cancer, and/or likelihood of metastasis in a patient susceptible to or diagnosed with a cancer. Prognosis also includes prediction of favorable responses to cancer treatments, such as a conventional cancer therapy. A response may be either a therapeutic response (sensitivity or recurrence-free survival during or after a treatment) or a lack of therapeutic response (residual disease, which may indicate resistance or recurrence during or after a treatment).

By "subject" or "patient" is meant any single subject for which therapy is desired, including humans, cattle, dogs, guinea pigs, rabbits, chickens, and so on. Also intended to be included as a subject are any subjects involved in clinical research trials not showing any clinical sign of disease, or subjects involved in epidemiological studies, or subjects used as controls.

As used herein, "increased expression" or "overexpression" or "decreased expression" refers to an expression level of a gene in the subject's sample as compared to a reference level representing the same gene or a different gene. In certain aspects, the reference level may be a reference level of expression from a non-cancerous tissue from the same subject. Alternatively, the reference level may be a reference level of expression from a different subject or group of subjects. For example, the reference level of expression may be an expression level obtained from a sample (e.g., a tissue, fluid or cell sample) of a subject or group of subjects without cancer, or an expression level obtained from a non-cancerous tissue of a subject or group of subjects with cancer. The reference level may be a single value or may be a range of values. The reference level of expression can be determined using any method known to those of ordinary skill in the art. In some embodiments, the reference level is an average level of expression determined from a cohort of subjects with cancer or without cancer or include both. The reference level may also be depicted graphically as an area on a graph. In certain embodiments, a reference level is a normalized level, a median, an average value, while in other embodiments, it may be a level that is not stable with respect to the tissue or biological sample being tested.

"About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

III. Nucleic Acid AssaysAspects of the methods include assaying nucleic acids to determine expression levels. Arrays can be used to detect differences between two samples. An array comprises a solid support with nucleic acid probes attached to the support. Arrays typically comprise a plurality of different nucleic acid probes that are coupled to a surface of a substrate in different, known locations. These arrays, also described as "microarrays" or colloquially "chips" have been generally described in the art, for example, U.S. Pat. Nos. 5,143,854, 5,445,934, 5,744,305, 5,677,195, 6,040,193, 5,424,186 and Fodor et al., 1991), each of which is incorporated by reference in its entirety for all purposes. Techniques for the synthesis of these arrays using mechanical synthesis methods are described in, e.g., U.S. Pat. No. 5,384,261, incorporated herein by reference in its entirety for all purposes. Although a planar array surface is used in certain aspects, the array may be fabricated on a surface of virtually any shape or even a multiplicity of surfaces. Arrays may be nucleic acids on beads, gels, polymeric surfaces, fibers such as fiber optics, glass or any other appropriate substrate, see U.S. Pat. Nos. 5,770,358, 5,789,162, 5,708,153, 6,040,193 and 5,800,992, which are hereby incorporated in their entirety for all purposes.

In addition to the use of arrays and microarrays, it is contemplated that a number of difference assays could be employed to analyze genes, their expression and activities, and their effects. Such assays include, but are not limited to, nucleic acid amplification, polymerase chain reaction, quantitative PCR, RT-PCR, RNA sequencing (e.g., by next-generation sequencing techniques), in situ hybridization, Northern hybridization, hybridization protection assay (HPA) (GenProbe), branched DNA (bDNA) assay (Chiron), rolling circle amplification (RCA), single molecule hybridization detection (US Genomics), Invader assay (ThirdWave Technologies), and/or Bridge Litigation Assay (Genaco).

The term "primer," as used herein, is meant to encompass any nucleic acid that is capable of priming the synthesis of a nascent nucleic acid in a template-dependent process. In particular embodiments, primers are oligonucleotides from ten to twenty and/or thirty base pairs in length, but longer sequences can be employed. Primers may be provided in double-stranded and/or single-stranded form, although the single-stranded form is preferred.

### IV. Kits

In various aspects, a kit is envisioned containing one or more oligonucleotides or other reagents described herein. The kit may contain one or more sealed containers, such as a vial, containing any of the reagents described herein and/or reagents for preparing any of the reagents described herein. In some embodiments, the kit may also contain a suitable container means, which is a container that will not react with components of the kit, such as an Eppendorf tube, an assay plate, a syringe, a bottle, or a tube. The container may be made from sterilizable materials such as plastic or glass.

The kit may further include instructions that outline the procedural steps for carrying out the diagnostic, treatment, or prevention of disease, and will follow substantially the same procedures as described herein or are known to those of ordinary skill. The instruction information may be in a computer readable media containing machine-readable instructions that, when executed using a computer, cause the display of a real or virtual procedure of using the kit described herein.

The kit can further comprise reagents for labeling mRNA of genes to be measured in the sample. The kit may also include labeling reagents, including at least one of amine-modified nucleotide, poly(A) polymerase, and poly(A) polymerase buffer. Labeling reagents can include an amine-reactive dye.

In further embodiments, a kit is provided comprising oligonucleotides that bind or are capable of hybridizing, respectively, to two, three, four, five or six genes chosen from the group of PARI, BLM, RAD51, RIF1, BRCA1, Ku80, RAD51AP1, RAD54B, Plk1, BRCA2, RAD51C, PALB2, MYC, and STAT3. In some embodiments, the kit includes oligonucleotides that bind or are capable of hybridizing, respectively, to two, three, four, five, six, seven or eight genes chosen from the group consisting of PARI, RIF1, Ku80, RAD51, BLM, BRCA1, RAD51AP1 and RAD54B. In particular embodiments, the kit includes oligonucleotides capable of hybridizing, respectively, to two, three, four, five, six, seven or eight genes chosen from the group consisting of RIF1, PARI, RAD51, Ku80, MYC and STAT3. In particular embodiments, the kit includes one or more oligonucleotides capable of hybridizing to a RIF1 gene sequence, one or more oligonucleotides capable of hybridizing to a PARI gene sequence, one or more oligonucleotides capable of hybridizing to a RAD51 gene sequence, and one or more oligonucleotides capable of hybridizing to a Ku80 gene sequence.

In certain embodiments, the oligonucleotides may be, be at least, or be at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, or 300 nucleotides, or any range derivable therein, in length. Each oligonucleotide may be a probe or primer that is labeled or un-labeled, and can hybridize under stringent hybridization conditions to an mRNA or cDNA encoded by one of the genes, or may be part of or full-length cDNA encoded by one of the genes, or may be part of or full-length cDNA encoded by one of the genes.

The kits may be used in detecting gene expression in cells or tissue from a patient, particularly tumor tissues from a cancer patient. For example, in real-time TaqMan PCR, oligonucleotides may be used as PCR primers, and/or TaqMan probe. In sequencing, the oligonucleotides may PCR primers and/or sequencing primers. In next-generation sequencing, the oligonucleotides may be used as capturing probes to capature targeted genes or mRNAs or cDNAs. In microarray-based gene expression profiling, the oligonucleotides may be probes attached to a solid support forming a hybridization chip.

Thus, the kits may additional include one or more reagents useful for PCR reactions, sequencing reactions, and/or hybridization reactions, such as Taq polymerase, reaction buffers, dNTPs, etc.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the claims.

### EXAMPLE 1. The RPS system was developed using data from carcinoma cell lines.

The inventors sought to create a method that predicts the efficiency of HR repair within any given cancer. To accomplish this, the inventors developed a scoring system that correlates gene expression patterns with HR proficiency in human cancer cell lines. Gene expression levels and corresponding drug sensitivity data were collected from the Broad-Novartis Cancer Cell Line Encyclopedia (CCLE) (Barretina, et al., 2012). Given the wide biological diversity known to exist between different classes of human malignancies, the inventors limited this analysis to cell lines derived only from carcinomas. Cellular resistance to the topoisomerase-I inhibiting drug topotecan was selected as a surrogate marker for HR proficiency. Topotecan is a derivative of camptothecin, and this class of drugs was selected because it disrupts replication forks and exerts toxicity preferentially in cells that harbor HR defects (Nitiss, et al., 1988; Arnaudeau, et al., 2001). Topotecan sensitivity data were available for 279 of the 634 carcinoma cell lines.

To focus the inventors analysis on the primary cellular features that mediate specific phenotypes, the inventors restricted the analysis to genes with direct relevance to replication stress and the DSB repair pathways **(****Figure 1****)**. The inventors further limited the analysis to 33 central proteins that participate in cellular preference toward HR vs. NHEJ, following the ataxia telangiectasia mutated (ATM) and/or ataxia telangiectasia and Rad3-related protein (ATR) activation steps of DNA damage response. Levels of mRNA were available for all of these genes except Ku70. Secondary regulators of damage response (like TP53, PTEN, and cell cycle checkpoint genes) were not considered as gene candidates for the scoring system, since they exert cellular influences that extend beyond the scope of replication stress and DSB repair. Pearson's correlation analyses demonstrated that 12 of the final list of 32 candidate genes had expression levels that significantly correlated (defined as p<0.05) with cellular sensitivity to topotecan **(Table 1)**. In all 12 cases, increasing gene expression levels directly correlated with increasing topotecan sensitivity.

**Table 1 - DNA repair genes that significantly associate with topotecan sensitivity.**

| A) Significant Genes | | | |
|---|---|---|---|
| | | | |

| Genes known to antagonize HR | HR-related genes | Correlation Coeff. | p-value |
|---|---|---|---|
| PARI | | -0.23 | <0.005 |
| | RAD51 | -0.18 | <0.005 |
| | BLM | -0.18 | <0.005 |
| RIF1 | | -0.17 | 0.005 |
| Ku80 | | -0.16 | 0.006 |
| | BRCA1 | -0.16 | 0.006 |
| | RAD51AP1 | -0.16 | 0.007 |
| | RAD54B | -0.15 | 0.02 |
| | Plk1 | -0.14 | 0.02 |
| | BRCA2 | -0.14 | 0.02 |
| | RAD51C | -0.13 | 0.03 |
| | PALB2 | -0.12 | 0.04 |
| | | | |

| B) Non-significant Genes | | | |
|---|---|---|---|
| | | | |

| Genes | Correlation Coeff. | p-value | |
|---|---|---|---|
| CtIP | -0.11 | 0.06 | |
| RAD51B | -0.11 | 0.06 | |
| DNA-PKcs | -0.10 | 0.09 | |
| PIAS4 | -0.09 | 0.15 | |
| XRCC3 | -0.08 | 0.16 | |
| XRCC2 | -0.08 | 0.18 | |
| RAD52 | -0.07 | 0.25 | |
| XRCC4 | -0.07 | 0.27 | |
| Artemis | -0.06 | 0.29 | |
| RAD51D | -0.06 | 0.30 | |
| WRN | -0.06 | 0.30 | |
| RAD54L | -0.06 | 0.33 | |
| HELQ | -0.04 | 0.47 | |
| MDC1 | -0.02 | 0.70 | |
| LIG4 | -0.02 | 0.70 | |
| PIAS1 | -0.02 | 0.72 | |
| Fbh1 | -0.01 | 0.90 | |
| RNF8 | 0.01 | 0.89 | |
| RNF4 | 0.01 | 0.83 | |
| TP53BP1 | 0.05 | 0.45 | |

### EXAMPLE 2 - HR-related genes are highly expressed in cancer cells that harbor low HR efficiency.

RIF1, Ku80, and PARI were among the genes whose expression most strongly associated with topotecan sensitivity. RIF1 and Ku80 are known to promote NHEJ and antagonize HR (Zimmermann, et al., 2013; Chapman, et al., 2013; Bennardo, et al., 2008). PARI is a helicase capable of disrupting RAD51 nucleofilaments, and it has been reported to antagonize HR repair (Moldovan, et al., 2012).

Topotecan sensitivity also correlated with the overexpression of a family of HR-related genes, including RAD51, BLM, BRCA1, RAD51-AP1, RAD54B, PLK1, BRCA2, RAD51C, and PALB2. This observation appears counterintuitive on the surface, since RAD51 and many of these RAD51-associated proteins are generally considered to promote HR. However, RAD51 overexpression has been previously shown to occur in the setting of HR defects caused by BRCA mutations (Martin, et al., 2007; Honrado, et al., 2005). To investigate a possible connection between BRCA mutation phenotypes and the inventors observed expression patterns, the inventors analyzed gene expression levels in CCLE cell lines that harbor BRCA1 (HCC1937 and MDA-MB436) or BRCA2 (CAPAN1) mutations. Consistent with published observations in BRCA1-mutant human tumors (Martin, et al., 2007), these three cell lines significantly overexpressed RAD51 and RAD51AP1. In addition, the inventors found that BRCA-defective cells significantly overexpress additional genes known to promote various mechanisms required for HR, including CtIP which promotes the 5' to 3' ssDNA end resection (Sartori, et al., 2007), Plk1 which promotes the phosphorylation of 53BP1 and RAD51 (Yata, et al., 2012; van Vugt, et al., 2010), and several genes (XRCC2, XRCC3, PALB2) that promote RAD51 filament assembly (Thompson, et al., 2012). These data suggest that BRCA-defective cells respond to their HR defects by increasing the expression of a fairly broad array of HR-related genes. The overexpression of HR genes as a compensatory mechanism has been proposed previously, particularly since RAD51 overexpression is known to partially suppress the HR defects that occur when key HR genes are mutated (Martin, et al., 2007; Takata, et al., 2001).

These findings were used to refine the list of genes to be used in the Recombination Proficiency Score (RPS). The inventors hypothesized that when HR-deficiency occurs in wild type BRCA backgrounds, cells respond via compensatory overexpression of HR-related genes that mirrors the phenotypes observed in BRCA mutant cells. As such, the inventors reasoned that many of the HR-related genes were reporting redundant predictive information in response to low HR proficiency. Gene expression levels were combined to generate a single model that predicts topotecan sensitivity, starting with genes that have known HR-antagonizing activities (RIF1, Ku80, and PARI) in order of their independent predictive power (**Table 2**). The family of HR-related genes was then subsequently added incrementally into this model. The addition of RAD51 improved the model's correlation with topotecan sensitivity (relative to the initial 3 genes), however the inclusion of additional HR-related genes did not further improve the correlation. Therefore, the final four genes selected to derive the RPS were Rif1, PARI, Ku80, and RAD51.

**Table 2 - Correlation coefficients Significant DNA repair genes from Table 1A were combined to determine the optimal number of genes, wherein sum of their expression levels correlated most strongly with topotecan sensitivity.**

| Combinations of genes | Correlation Coeff. |
|---|---|
| PARI | -0.23 |
| PARI + RIF1 | -0.25 |
| PARI + RIF1 + Ku80 | -0.26 |
| **PARI + RIF1 + Ku80 + RAD51** | **-0.28** |
| PARI + RIF1 + Ku80 + RAD51 + BLM | -0.27 |
| PARI + RIF1 + Ku80 + RAD51 + BLM + BRCA1 | -0.27 |
| PARI + RIF1 + Ku80 + RAD51 + BLM + BRCA1 + RAD51AP1 | -0.26 |
| PARI + RIF1 + Ku80 + RAD51 + BLM + BRCA1 + RAD51AP1 + RAD54B | -0.26 |

Elevated mRNA levels for any of these genes correlated with greater sensitivity to topotecan. The RPS was defined as the sum of these four expression levels multiplied times -1, using the log2 transformed mRNA values of each gene normalized to the median mRNA within the starting 634 carcinoma cell lines. The median RPS score within the carcinoma cell lines was approximately zero, the bottom 25^{th} percentile of RPS scores were less than -1.08, and the top 25^{th} percentile of RPS scores were greater than 1.2.

Interestingly, CCLE cell lines with low RPS scores did indeed overexpress a broad array of HR-related genes **(****Figure 2****)**. These data support the existence of a compensatory mechanism that responds to low HR efficiency. Furthermore, these results suggest that this compensatory mechanism is not limited to only the most extreme HR defects, like those resulting from BRCA mutations. MEN1 protein was considered as a possible mediator of this proposed compensatory process, since MEN1 has been shown to stimulate the transcription of several HR genes, including BRCA1, RAD51, and RAD51AP1 (Fang, et al., 2013). However this explanation was deemed unlikely, since MEN1 mRNA levels did not significantly correlate with RPS in CCLE cell lines.

### EXAMPLE 3 - RPS predicts HR proficiency in individual cancer cell lines.

The predictive value of the RPS was further tested based on sensitivity to different types of chemotherapeutic agents. Similar to results with topotecan, low RPS scores correlated to sensitivity to irinotecan, another topoisomerase-I inhibiting drug **(****Figure 3A****)**. This is expected, since topoisomerase-I inhibitors generate replication fork disruptions, which require HR for repair (Nitiss, et al., 1988; Arnaudeau, et al., 2001). As a control, this analysis was repeated using the non-DNA damaging drug paclitaxel, and RPS did not show a correlation with sensitivity to this agent. These results support the specificity of RPS to DNA-related damage and repair. It should be noted that complete drug sensitivity data was not available for all three chemotherapy agents in all cell lines evaluated (see **FIG 7** for breakdown). However, comparable results were observed when the analyses were repeated on the subset of 137 cell lines that were tested with all three agents.

The ability of RPS to predict repair pathway preference was further tested by measuring HR repair efficiency in representative cell lines with low RPS (RKO, DU 145, COLO 205) or with mid/high RPS (PC3, HCC44, NCI-H650). These cell lines exhibited expected levels of sensitivity to topotecan and paclitaxel when independently re-tested in the inventors laboratory (**FIG 8**), which were comparable to the sensitivities mined from the CCLE database. These six cell lines were tested using a modified version of the previously described DR-GFP reporter method (Pierce, et al., 1999). This method utilizes a reporter DNA construct that carries two non-functional copies of green fluorescence protein (GFP), one of which is interrupted by an I-SceI endonuclease site. Induction of a DSB at the I-SceI site can lead to repair by homologous gene conversion that generates a functional copy of GFP. As demonstrated in **Figure 3B**, RPS correlated with HR efficiency on linear regression analysis (R²=0.833, two-sided *p*=0.003). For consistency with the other results, RPS values for these cells were calculated using array-based mRNA levels from the CCLE database. The inventors verified the identity of the inventors six cell lines by short tandem repeat profiling (Genetic Resources Core Facility at Johns Hopkins School of Medicine), and independent quantitation by real time qRT-PCR generated mRNA measurements that were comparable to the mRNA levels reported in the CCLE database (**FIG 9**).

### EXAMPLE 4 - Cell lines with high RPS have elevated genomic instability.

HR plays a central role in maintaining genomic stability in cells. The inventors hypothesized that cells with low RPS would exhibit more genome instability than cells with high RPS. To test this hypothesis, SNP array-based DNA copy number variations (CNVs) were analyzed using CCLE carcinoma cell lines **(****Figure 4****)**. Low RPS scores were associated with more frequent DNA amplifications. This finding is consistent with published analyses of HR-defective cell lines, showing that mutations in RAD51D or XRCC3 promote DNA amplifications (Hinz, et al., 2006). These amplifications are proposed to result from stress-induced replication fork disruption and subsequent homology-mediated polymerase template switching (Arlt, et al., 2012; Malkova, et al., 2012). A study in RAD51 defective S. cerevisiae demonstrated that cells with deregulated HR frequently channel DSBs into repair by non-allelic break-induced replication, thereby stimulating the formation of segmental duplications (Payen, et al., 2008). Additionally, the inventors found that cells with low RPS harbored relatively frequent DNA deletions. Deletions are characteristic of error prone repair processes like microhomology-mediated end joining and single-strand annealing (Guirouilh-Barbat, et al., 2004; Bennardo, et al., 2008). Of note, the distributions of CNV sizes were not strongly influenced by RPS. Taken together, these results suggest that low RPS cells have reduced HR proficiency and rely more on error-prone processes to rejoin DSBs and/or to tolerate replication stress.

Mutations in TP53 are also known to exert major influences on cellular resistance to DNA damaging therapies and genomic instability. Additionally, TP53 mutation status has been shown to influence HR efficiency (Linke, et al., 2003; Sirbu, et al., 2011). Therefore, the inventors re-examined RPS -associated CNVs in the context of TP53 mutation status. The average RPS was not significantly different between the 238 TP53 WT cell lines and the 386 TP53 mutant cell lines (0.25 vs. 0.41, *p*=0.41). Also, the association between increased CNVs and low RPS was observed in both TP53 WT and mutant cell line groups. The magnitude of RPS dependence was less pronounced in TP53 mutant cells, due to a high background of deletions in TP53 mutant cells. A high deletion frequency is not surprising in TP53 mutant cells, since deletions are known to occur 40-300 times more following TP53 inactivation (Gebow, et al., 2000). These data suggest, therefore, that TP53 mutation status and RPS offer independent predictive information regarding genomic instability.

A possible relationship between RPS and TP53 status was further studied by examining resistance to DNA damage. The ability of RPS to associate with topotecan sensitivity on logistic regression was similar in both TP53 WT and mutant cell line subgroups (*p<* 0.003 for both). This association supports the role of RPS as a predictor of HR proficiency, which is distinct from TP53-dependent activities like apoptotic threshold modulation and cell cycle regulation.

### EXAMPLE 5 - Human tumors with low RPS exhibit unfavorable clinical characteristics and elevated genomic instability.

The RPS system was clinically validated using tumor datasets from the Cancer Genome Atlas (CGA). Breast and non-small cell lung cancer (NSCLC) tumor types were selected for this analysis, because these datasets contained large sample sizes, annotations of clinical features, SNP array-based DNA CNV data, and adequate details on patient outcomes. Although some differences existed between different cancer types, tumors with lower RPS generally exhibited adverse clinical characteristics (**Table 3**). Low RPS tumors tended to be more locally/regionally advanced and to harbor more frequent TP53 mutations. For example, the lower quartile RPS tumors were significantly more likely to have lymph node invasion in non-small cell lung cancers (*p*=0.008). Similarly, breast cancers with low RPS commonly exhibited estrogen receptor loss (*p*=0.0001) and HER2 amplification (*p*=0.007).

**Table 3 Low RPS is associated with adverse clinical features in human tumors**

| | **RPS Quartile** | | | | |
|---|---|---|---|---|---|
| **Prognostic Factor** | **0-25th** | **25-50th** | **50-75th** | **75-100th** | ***p*-value** |
| | | | | | |

| **Non-small cell lung cancer** | | | | | |
|---|---|---|---|---|---|
| T3/4 tumor | 16% | 11% | 14% | 8% | 0.75 |
| Lymph node invasion | 51% | 33% | 33% | 14% | 0.0085 |
| TP53 mutation | 89% | 75% | 86% | 44% | <0.0001 |
| | | | | | |

| **Breast cancer** | | | | | |
|---|---|---|---|---|---|
| T3/4 tumor | 11% | 16% | 10% | 16% | 0.66 |
| Lymph node invasion | 48% | 59% | 58% | 46% | 0.29 |
| TP53 mutation | 60% | 40% | 39% | 19% | <0.0001 |
| Estrogen receptor loss | 46% | 23% | 17% | 6% | <0.0001 |
| HER2 amplification | 14% | 19% | 19% | 0% | 0.0072 |

| | | | | | |
|---|---|---|---|---|---|
| *p*-values denote differences in frequencies among groups based on a likelihood ratio test | | | | | |

These adverse features associated with low RPS may be the result of low-fidelity repair processes, which in turn promote genomic instability and malignant progression. To explore this hypothesis the inventors analyzed CNV as a function of RPS using these same two CGA tumor datasets. Both carcinoma types exhibited at least one class of elevated CNV in the setting of low RPS (**Figure 5**). This RPS -associated genome instability was observed in both TP53 WT and mutant tumors. These results suggest that mutagenic DNA repair processes dominate in low RPS tumors, thereby promoting the evolution of malignant clinical features.

### EXAMPLE 6 - RPS is prognostic and predictive of treatment sensitivity in clinical tumors.

Next the inventors evaluated whether RPS can predict clinical outcomes in human tumors. NSCLC was considered an appealing tumor type for this analysis, since NSCLC-directed chemotherapy regimens are generally platinum-based and since lung cancer is a leading cause of cancer mortality. The inventors also sought to distinguish the prognostic and predictive utilities of RPS. Specifically, the inventors hypothesized that low RPS would confer a poor prognosis, because of elevated mutagenesis and associated adverse tumor features. However, the inventors also hypothesized that sensitivity to platinum-based chemotherapeutic agents is expected to simultaneously render low RPS tumors treatment-sensitive, given that HR-defective cells are hypersensitive to DNA cross-linkers. These opposing effects were predicted to counteract one another in low RPS tumors treated with chemotherapy.

The power of RPS to predict outcome in NSCLC patients was investigated using data from the JBR.10 clinical trial, which had previously demonstrated a benefit to adjuvant chemotherapy in early-stage NSCLC (Zhu, et al., 2010). Specifically, JBR.10 had randomly assigned patients to receive cisplatin + vinorelbine chemotherapies vs. no further treatment, following the resection of stage I-II NSCLCs. This dataset was ideal for the inventors analysis because of its prospective randomized trial design, combined with uniform treatment details. As such, it does not suffer from the biases intrinsic to retrospectively collected datasets. In patients whose treatment consisted of surgery only, low RPS predicted for inferior 5-year overall survival relative to higher RPS (15% vs. 60%, *p* = 0.004, log rank test). This clinically validates the prognostic power of RPS (**Figure 6A**). Chemotherapy significantly improved 5-year overall survival in low RPS tumors (15% vs. 77%, *p* = 0.01) but did not in high RPS tumors (60% vs. 72%, *p* = 0.55). This clinically validates the ability of RPS to predict sensitivity to platinum-based chemotherapy.

These data suggest that the poor prognoses associated with low RPS might be negated by chemotherapy, since low RPS tumors are especially sensitivity to platinum-based chemotherapy. In the JBR.10 trial, for example, patients treated with chemotherapy had similar 5-year overall survival rates regardless of low vs. higher RPS (77% vs. 72%, *p* = 0.70). To study this further, the inventors selected three additional datasets containing retrospectively collected data on NSCLC patients (Okayama, et al., 2012; Tang, et al., 2013; Shedden, et al., 2008). After controlling for stage on multivariate analysis, low RPS was again associated with poor survival in patients treated with surgery alone (**Figure 6B**). Specifically, the inventors combined data from all four datasets using previously described methodology (Neyeloff, et al., 2012) and found that low RPS confers a continuous hazard ratio of 1.24 (95% CI = 1.12- 1.36). When this analysis was repeated on patients treated with surgery plus adjuvant chemotherapy, the poor prognosis associated with low RPS was diminished (hazard ratio = 0.94, 95% CI = 0.69-1.21). Taken together, these findings support the hypothesis that patients with low RPS tumors have adverse underlying prognoses, but that HR suppression and associated sensitivity to platinum-based drugs counteracts these adverse prognostic features. Therefore, RPS may help oncologists select which therapies will be effective for individual patients, thereby enabling more personalized care.

### EXAMPLE 7 - Materials And Methods

*Study Design:* The inventors sought to create a method that predicts the efficiency of HR repair using publically available data on human cancer cell lines. Specifically, the inventors developed a scoring system that correlates gene expression patterns with HR proficiency. Data for mRNA expression, copy number variation, and drug sensitivity for human carcinoma cell lines (n=634) were collected from the Broad-Novartis Cancer Cell Line Encyclopedia (CCLE). Robust Multi-array Average (RMA)-normalized mRNA expression values were normalized to the median value across all carcinoma samples and subsequently log2 transformed. SNP array-based DNA copy number values were filtered to eliminate individual SNPs. For CNV analysis, minimum deletion size was defined as copy number segment mean ≤ -0.6, while minimum insertion size was defined as copy number segment mean ≥ +1.4 (log2 [copy number/2]). Deletions and insertions were binned by size, whereby bins represent 10-fold increments in size. Drug sensitivities for topotecan (n=279), irinotecan (n=180), and paclitaxel (n=257) were determined by IC₅₀ values. IC₅₀ values ≥ 8 µM were outliers and, therefore, censored from the analysis. TP53 mutation status was determined by hybrid capture sequencing data, which was available for all carcinoma cell lines. In the six cell lines used for HR reporter experiments (RKO, DU 145, COLO 205, PC3, HCC44, NCI-H650), sensitivities to topotecan and paclitaxel were confirmed in the inventors lab using an acute continuous 3-day exposure of cells to drugs; this method is identical to the method that was used to generate the CCLE drug sensitivity data.

*Quantification of HR efficiency in cells:* An HR reporter-containing plasmid (pDR-GFP), an I-Sce I expressing plasmid (pCβASce), and an empty vector control plasmid (pCAG) were provided by Maria Jasin. Cells transiently co-transfected with combinations of either pDR-GFP + pCβASce or pDR-GFP + pCAG. To accomplish this, 0.5 x 10⁶ cells at 80% confluence were electroporated with 15 µg of each plasmid in 4mm cuvettes, using the following settings: 325-375 V, 975 µF. Electroporation voltages were optimized in order to minimize differences in transfection efficiencies between the six cell lines. Cells were transferred into the appropriate complete growth medium and allowed to grow for 48 hours, following which they were analyzed with a Becton-Dickinson FACScan. Live cells were collected based on size/complexity and 7-aminoactinomycin D (7-AAD) exclusion. The fraction of live cells exhibiting GFP positivity was quantified. To account for any remaining differences that persisted in transfection efficiencies between cell lines, the GFP positivity resulting from pDR-GFP + pCβASce transfection was normalized to GFP positivity resulting from pDR-GFP + pCAG transfection. Experiments were performed in triplicate, and the displayed error bars denote standard error.

*Evaluation of RPS in human tumor datasets and association with clinical characteristics:* Breast and non-small cell lung cancer (NSCLC) tumor datasets were collected from the Cancer Genome Atlas (CGA). Stage IV patients with metastatic disease or those patients without a specified stage were excluded from analysis. TP53 mutation status was determined by SNP array-based DNA copy number data. Normalized mRNA expression, copy number variation, and TP53 mutation status were available for 295 breast cancers and 153 NSCLCs. CNV analysis was performed as described for the CCLE carcinoma cell lines. Clinical characteristics and prognostic factors were available for 280 breast cancers and 145 NSCLCs with available mRNA expression data.

*Validation of the RPS system using clinical databases:* Four publicly available NSCLC datasets were collected from Gene Expression Omnibus (GEO; accession numbers GSE14814 [JBR.10 trial], GSE31210 [Japanese National Cancer Center Research Institute], and GSE42127 [MD Anderson Cancer Center]) and from the National Cancer Institute caArray website at https://array.nci.nih.gov/caarray/project/jacob-00182 [Director's Challenge Consortium]. mRNA expression values were normalized to the median value across all patient samples within each respective dataset and subsequently log2 transformed. Patient samples were grouped based on type of treatment. In total, 581 patients underwent surgery alone and 164 patients received surgery + chemotherapy. Cox proportional hazard analysis for overall survival was used to determine the hazard ratio for the RPS as a continuous variable. All NSCLC dataset analyses were limited to stage I and II patients.

*Statistical analysis:* All analyses were performed using JMP 9.0 (SAS Institute Inc.; Cary, NC). A p-value ≤ 0.05 was considered statistically significant.

All of the methods and apparatuses disclosed and claimed herein can be made and executed without undue experimentation in light of the present disclosure. While the compositions and methods of this invention have been described in terms of preferred embodiments, it will be apparent to those of skill in the art that variations may be applied to the methods and apparatuses and in the steps or in the sequence of steps of the methods described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references to the extent that they provide exemplary procedural or other details supplementary to those set forth herein, are specifically incorporated herein by reference.
Arlt, et al., PLoS Genet 8, e1002981 (Sep, 2012).
Arnaudeau, et al., J Mol Biol 307, 1235 (Apr 13, 2001).
Barretina et al., Nature 483, 603 (Mar 29, 2012).
Bennardo, et al., PLoS Genet 4, e1000110 (Jun, 2008).
Biedermann, et al., Proc Natl Acad Sci USA 88, 1394 (Feb 15, 1991).
Bindra, et al., Oncogene 26, 2048 (Mar 29, 2007).
Birkelbach et al., J Thorac Oncol 8, 279 (Mar, 2013).
Bryant et al., Nature 434, 913 (Apr 14, 2005).
Buis, et al., Nat Struct Mol Biol 19, 246 (Feb, 2012).
Carter, et al., Nat Genet 38, 1043 (Sep, 2006).
Chapman et al., Mol Cell 49, 858 (Mar 7, 2013).
Chapman, et al., J Cell Sci 125, 3529 (Aug 1, 2013).
Edwards et al., Nature 451, 1111 (Feb 28, 2008).
Fang et al., Mol Cell Biol, (May 6, 2013).
Farmer et al., Nature 434, 917 (Apr 14, 2005).
Flygare et al., Exp Cell Res 268, 61 (Aug 1, 2001).
Fulop, et al., Nature 347, 479 (Oct 4, 1990).
Galanty et al., Nature 462, 935 (Dec 17, 2009).
Galanty, et al., Genes Dev 26, 1179 (Jun 1, 2012).
Gebow, et al., Mol Cell Biol 20, 4028 (Jun, 2000).
Guirouilh-Barbat et al., Mol Cell 14, 611 (Jun 4, 2004).
Hinz et al., Nucleic Acids Res 34, 1358 (2006).
Honrado et al., J Clin Oncol 23, 7503 (Oct 20, 2005).
Kim, et al., Nucleic Acids Res 29, 4352 (Nov 1, 2001).
Liauw, et al., Sci Transl Med 5, 173sr2 (Feb 20, 2013).
Lieber, et al., DNA Repair (Amst) 3, 817 (Aug-Sep, 2004).
Linke et al., Cancer Res 63, 2596 (May 15, 2003).
Malkova, et al., Annual review of genetics 46, 455 (2012).
Martin et al., Cancer Res 67, 9658 (Oct 15, 2007).
Moldovan et al., Mol Cell 45, 75 (Jan 13, 2012).
Mortensen, et al., Proc Natl Acad Sci USA 93, 10729 (Oct 1, 1996).
Neyeloff, et al., BMC Res Notes 5, 52 (2012).
Nitiss, et al., Proc Natl Acad Sci U S A 85, 7501 (Oct, 1988).
Okayama et al., Cancer Res 72, 100 (Jan 1, 2012).
O'Shaughnessy et al., N Engl J Med 364, 205 (Jan 20, 2011).
Payen, et al., PLoS Genet 4, e1000175 (2008).
Pierce, et al., Genes Dev 13, 2633 (1999).
Raderschall et al., Cancer Res 62, 219 (Jan 1, 2002).
Redon et al., Biochim Biophys Acta 1819, 743 (Jul, 2012).
Richardson, et al., Oncogene 23, 546 (Jan 15, 2004).
Sakai et al., Nature 451, 1116 (Feb 28, 2008).
Sarasin, et al., Mutat Res 659, 49 (Jul-Aug, 2008).
Sartori et al., Nature 450, 509 (Nov 22, 2007).
Shah et al., Mol Cell 39, 862 (Sep 24, 2010).
Shedden et al., Nat Med 14, 822 (Aug, 2008).
Shrivastav, et al., Cell Res, (Dec 24, 2008).
Sirbu et al., PLoS One 6, e23053 (2011).
Steensma, N Engl J Med, (May 1, 2012).
Takata et al., Mol Cell Biol 21, 2858 (2001).
Tang et al., Clin Cancer Res 19, 1577 (Mar 15, 2013).
Thompson, et al., Mutat Res 477, 131 (2001).
Thompson, Mutat Res 751, 158 (Oct-Dec, 2012).
van Vugt et al., PLoS Biol 8, e1000287 (Jan, 2010).
Wahba, et al., Elife 2, e00505 (2013).
Willers et al., Mol Cancer Res 7, 1304 (Aug, 2009).
Winnepenninckx et al., J Natl Cancer Inst 98, 472 (Apr 5, 2006).
Yata et al., Mol Cell 45, 371 (Feb 10, 2012).
Zhu et al., J Clin Oncol 28, 4417 (Oct 10, 2010).
Zimmermann, et al., Science 339, 700 (Feb 8, 2013).

## Claims

1. A composition comprising a platinum compound, a DNA cross-linking agent, a topoisomerase inhibitor or a PARP inhibitor for use in treating cancer in patients tested to carry tumor cells or tissue with homologous recombination repair deficiency, and wherein said deficiency is tested by:
determining the expression level of the i) PARI, RIF1, Ku80, RAD51, BLM and BRCA1 genes, or ii) of the RAD51 gene and one or both of the Ku80 and BRCA1 genes in tumor cells or tissue;
calculating a recombination proficiency score (RPS) using the determined gene expression level; and
comparing the calculated RPS score to a reference score, wherein said RPS score being lower than said reference score would indicate said tumor cells or tissue have homologous recombination repair deficiency and are sensitive to the platinum compound, DNA cross-linking agent, topoisomerase inhibitor and PARP inhibitor.

2. The composition for the use of claim 1, wherein said calculating said recombination proficiency score (RPS) comprises combining the normalized gene expression level of the i) PARI, RIF1, Ku80, RAD51, BLM and BRCA1 genes (mRNA level), or ii) of the RAD51 gene and one or both of the Ku80 and BRCA1 genes (mRNA level) to arrive at a score.

3. The composition for the use of claim 1 or 2, wherein said determining the expression level of the i) PARI, RIF1, Ku80, RAD51, BLM and BRCA1 genes, or ii) of the RAD51 gene and one or both of the Ku80 and BRCA1 genes in tumor cells or tissue comprises RT-PCR and normalization with housekeeping genes.

4. Use of a composition comprising diagnostic reagents that include oligonucleotides capable of specifically hybridizing respectively to the i) PARI, RIF1, Ku80, RAD51, BLM and BRCA1 genes, or ii) to the RAD51 gene and one or both of the Ku80 and BRCA1 genes in a method of predicting therapeutic efficiency in the treatment of cancer by determining homologous recombination repair proficiency in cancer.

5. A composition comprising diagnostic reagents that include (i) multiple oligonucleotides capable of hybridizing to the PARI gene, multiple oligonucleotides capable of hybridizing to the RIF1 gene, multiple oligonucleotides capable of hybridizing to the Ku80 gene, multiple oligonucleotides capable of hybridizing to the BLM gene, and multiple oligonucleotides capable of hybridizing to the BRCA1 gene, or ii) multiple oligonucleotides capable of hybridizing to the RAD51 gene and multiple oligonucleotides capable of hybridizing to one or both of the Ku80 and BRCA1 genes, for use in a method of predicting therapeutic efficiency in the treatment of cancer by determining homologous recombination repair proficiency in cancer.

6. The composition for the use of claim 4 or 5, wherein the diagnostic reagents are used to determine the expression level of said genes in tumor cells or tissue, or wherein the diagnostic reagents are used to determine tumor sensitivity to DNA cross-linking agents or topoisomerase inhibitors, or wherein the diagnostic reagents are used to determine tumor sensitivity to PARP inhibitors or radiotherapy.

7. The composition for the use of any one of claims 4 or 5, wherein said oligonucleotides are labeled or unlabeled primers or probes, or wherein said oligonucleotides are of a length of 2 to 200 nucleotides.

8. An in vitro method for determining the homologous recombination repair proficiency in tumor cells or tissue comprising:
determining the expression level of the the i) PARI, RIF1, Ku80, RAD51, BLM and BRCA1 genes, or ii) of the RAD51 gene and one or both of the Ku80 and BRCA1 genes in tumor cells or tissue;
calculating a recombination proficiency score (RPS) using the determined gene expression level;
comparing the calculated RPS score to a reference score, wherein said RPS score being lower than said reference score would indicate a deficiency of homologous recombination repair in said tumor cells or tissue, and said RPS score being higher than said reference score would indicate a proficiency of homologous recombination repair in said tumor cells or tissue.

9. The method of claim 8, wherein said calculating said recombination proficiency score (RPS) comprises combining the normalized gene expression level of the the i) PARI, RIF1, Ku80, RAD51, BLM and BRCA1 genes, or ii) of the RAD51 gene and one or both of the Ku80 and BRCA1 genes (mRNA level) to arrive at a score, and/or wherein said determining the expression level of the i) PARI, RIF1, Ku80, RAD51, BLM and BRCA1 genes, or ii) of the RAD51 gene and one or both of the Ku80 and BRCA1 genes in tumor cells or tissue comprises RT-PCR and normalization with housekeeping genes.

10. The method of claim 8, wherein when the RPS score is lower than the reference score would indicate that the tumor cells or tissue are sensitive to radiation, platinum compounds, DNA cross-linking agents, and topoisomerase inhibitors, or wherein when the RPS score is lower than the reference score would indicate that the tumor cells or tissue are sensitive to PARP inhibitors.

11. A kit comprising:
an enclosed container; and
a composition including oligonucleotides capable of specifically hybridizing respectively to the i) PARI, RIF1, Ku80, RAD51, BLM and BRCA1 genes, or ii) to the RAD51 gene and one or both of the Ku80 and BRCA1 genes, and optionally further comprising a polymerase, reaction buffers and dNTPs for use in a method of predicting therapeutic efficiency in the treatment of cancer.

12. The kit for the use according to claim 11, wherein high expression of said genes would indicate that said tumor cells or tissue are sensitive to DNA cross-linking agents, topoisomerase inhibitors, PARP inhibitors or radiotherapy.

13. The kit for the use according to claim 11, comprising (i) multiple oligonucleotides capable of hybridizing to the PARI gene, multiple oligonucleotides capable of hybridizing to the RIF1 gene, multiple oligonucleotides capable of hybridizing to the Ku80 gene, multiple oligonucleotides capable of hybridizing to the BLM gene, and multiple oligonucleotides capable of hybridizing to the BRCA1 gene, or ii) multiple oligonucleotides capable of hybridizing to the RAD51 gene and multiple oligonucleotides capable of hybridizing to one or both of the Ku80 and BRCA1 genes.

14. The kit for the use of any one of claims 11-13, wherein said oligonucleotides are labeled or unlabeled primers or probes, or wherein said oligonucleotides are 20-200 nucleotides in length.

15. The composition for the use of any one of claims 4 to 7, wherein the diagnostic reagents are present in a kit which further comprises an enclosed container.
